(19) Europäisches Patentamt
European Patent Office
Office européen des brevets

(11) EP 0 856 146 B1

(12) EUROPÄISCHE PATENTSCHRIFT

(45) Veröffentlichungstag und Bekanntmachung des Hinweises auf die Patenterteilung:
21.07.2004 Patentblatt 2004/30

(21) Anmeldenummer: 96945356.2

(22) Anmeldetag: 16.10.1996

(51) Int Cl.$^{7}$: **G01F 1/704**, C21C 5/46

(86) Internationale Anmeldenummer:
**PCT/DE1996/001965**

(87) Internationale Veröffentlichungsnummer:
**WO 1997/015000 (24.04.1997 Gazette 1997/18)**

(54) **VERFAHREN ZUR BESTIMMUNG VON GASVOLUMENSTRÖMEN IN PROZESSEN DER FLÜSSIGPHASE IN EINEM ELEKTROOFEN**

METHOD FOR THE DETERMINATION OF GAS VOLUME STREAMS IN PROCESSES OF THE LIQUID PHASE IN AN ELECTRIC FURNACE

PROCEDE POUR DETERMINER DES FLUX DE VOLUMES DE GAZ DANS DES PROCESSUS DE LA PHASE LIQUIDE DANS UN FOUR ELECTRIQUE

(84) Benannte Vertragsstaaten:
**AT BE CH DE DK ES FI FR GB GR IE IT LI LU MC NL PT SE**

(30) Priorität: **17.10.1995 DE 19540225**

(43) Veröffentlichungstag der Anmeldung:
**05.08.1998 Patentblatt 1998/32**

(73) Patentinhaber: **Georgsmarienhütte GmbH**
**49124 Georgsmarienhütte (DE)**

(72) Erfinder:
• **PATEL, Pervez**
**D-49124 Georgsmarienhütte (DE)**
• **TREPPSCHUH, Frank**
**D-49134 Wallenhorst (DE)**
• **VAN HÜLLEN, Peter**
**D-52072 Aachen (DE)**

(74) Vertreter: **Bauer, Wulf, Dr.**
**Lindenallee 43**
**50968 Köln-Marienburg (DE)**

(56) Entgegenhaltungen:
**US-A- 3 934 470**

• **STAHL UND EISEN, Bd. 113, Nr. 6, 14.Juni 1993, DÜSSELDORF,DE, Seiten 55-60, XP000369701 KOEHLE S ET AL: "BEOBACHTUNG DES ENTKOHLUNGSPROZESSES ANHAND VON ABGASMESSUNGEN"**
• **J. Reichel et al "Beobachtung und Steuerung des Frischens hochchromhaltiger Stähle nach dem KCB-S-Verfahren", Stahl und Eisen, Vol. 113, Nr. 9, S.83, 1993**

## Beschreibung

Die vorliegende Erfindung betrifft ein Verfahren zur Bestimmung von direkter Messung nicht, oder nicht leicht zugänglicher Gasvolumenströme zu und von stahlerzeugenden Schmelzprozessen in einem Elektroofen,. der Undichtigkeiten aufweist.

Bekannt sind in jüngster Zeit Methoden zur Verringerung des elektrischen Energieverbrauchs bei Elektroöfen zur Stahlerzeugung, die vornehmlich die Nachverbrennung der primär entstandenen Reaktionsgase nutzen. Dabei sind spezifische Sauerstoffverbräuche von 30 - 40 $m^3/l$ erreicht worden - kaum weniger als für Konverterverfahren. Somit ergibt sich die Notwendigkeit von Modellen, die sowohl die thermischen, als auch die stofflichen Flüsse im Ofen beschreiben.

Die heutige Situation bei den Elektrostahlverfahren zeugt von einem eklatanten Mangel an erfolgreichen Prozeßmodellen, die in der Lage sind mittels einer Energie- und Massenbilanz die Ofenmetallurgie zu überwachen und zu steuern. Dieser Mangel ist darauf zurückzuführen, daß der Prozeß langsamer verläuft als beim Oxygenkonverter und daß die Möglichkeiten zur Temperatur- und Probenentnahme während der Chargenzeit weitaus besser sind als beim Konverterverfahren. Auch sind auf Prozeßsignalen basierende Verfahren zur Erzeugung von Stahl mittels Elektroofen Mangelware; so wird beispielsweise kaum eine Abgasanalyse durchgeführt, und auch die Abgasrate nicht gemessen.

Desweiteren ist die direkte Messung des Abgasvolumenstromes aufgrund seiner hohen Temperatur nicht bzw. nicht wirtschaftlich sinnvoll möglich. Eine langsame Abkühlung des Abgasstromes, die eine direkte Messung des Volumenstromes ermöglichen würde, scheidet darüberhinaus aus, da hierbei unerwünschte und umweltbelastende Dioxine freigesetzt werden können.

Darüber hinaus saugen Elektroöfen aufgrund von Undichtigkeiten unerwünscht Luft, die sogenannte Falschluft an, deren Volumenstrombestimmung sich ebenfalls einer direkten Messung entzieht.

Die US-A-3 934 470 offenbart eine Methode zur Messung der Volumenströme von aus einem Sauerstoffkonverter ausströmenden Abgasen. Dem eingeblasenen Sauerstoff wird Argon als Bestimmungsgas mitgegeben. Die Konzentration des. Argons wird in der dem Konverter entweichenden Abluft gemessen und hieraus der Volumenstrom des Abgases berechnet. Die Messung erfolgt an der Konvertermündung oder in der Abgasleitung. Berücksichtigt wird der Volumenstrom an Luft, der durch Undichtigkeiten zwischen Konvertermündung und einer Abgasleitung in die Abgasleitung eintritt.

In der DE-Veröffentlichung "Beobachtung des Entkohlungsprozesses ..." Köhle et al., Stahl und Eisen 113, Nr. 6 ist eine Methode zur Bestimmung der Entkohlung einer Stahlschmelze in einem Konverter aus dem im Abgas enthaltenden Reaktionsprodukten beschrieben. Der Eintrag von Sauerstoff durch die Falschluft wird berücksichtigt. Der Durchfluss der Falschluft wird durch eine Formel angegeben.

Der Erfindung liegt daher die Aufgabe zugrunde ein Verfahren zur Bestimmung von direkter Messung nicht, oder nicht leicht zugänglicher Gasvolumenströme der Falschluft und des Abgases zu und von stahlerzeugenden Schmelzprozessen in einem Elektroofen, der Undichtigkeiten aufweist, anzugeben.

Diese Aufgabe wird gelöst durch das Verfahren gemäß Patentanspruch 1. Vorzugsweise wird hierbei zur Messung der Bestimmungsgase (Tracer) in den dem Prozeß zuströmendem oder von diesem abströmenden Gasen ein Massenspektrometer benutzt wird. Die Analysegenauigkeit eines Massenspektrometers ist dabei wesentlich höher, die Analysezeit kürzer als. im Wege konventioneller Verfahren, die die Infrarotabsorption für CO und $CO_2$, die Wärmeleitfähigkeit für $H_2$ und die paramagnetische Suszeptibilität für $O_2$ verwenden und darüber hinaus auch noch einheitlich für alle Komponenten. Zudem können nur Massenspektrometer Inertgase wie Argon, Helium und Stickstoff analysieren. Das Massenspektrometer kann die Konzentration der Bestimmungsgase (Tracer) dabei in für die Prozeßführung geeigneten Intervallen messen.

Die Analyse des Abgases erfolgt hierbei vorzugsweise am Krümmer des Elektroofens.

Bei dem Verfahren nach der vorliegenden Erfindung strömen dem Schmelzprozeß im Elektroofen als Gase mindestens technischer Sauerstoff als gewollte Zufuhr mit einem quantitativ bekannten Volumenstrom und Umgebungsluft infolge von Undichtigkeiten des Elektroofens, als sogenannte Falschluft, mit quantitativ unbekanntem Volumenstrom zu und entströmen dem Prozeß darüberhinaus Abgase. Der Volumenstrom des technischen Sauerstoffs wird hierbei wie üblich direkt gemessen.

Vorzugsweise werden als Bestimmungsgase (Tracer) zwei Gase, nämlich das Inertgas Stickstoff ($N_2$) und das Edelgas Argon (Ar), die in bekannter oder zu messender Konzentration in den Gasen, die dem Prozeß zuströmen, nämlich in dem technischen Sauerstoff und in der Falschluft vorhanden sind oder diesen zugesetzt werden und die selbst durch den Prozeß keiner Veränderung, insbesondere keiner Verminderung oder Erhöhung ihres Volumens oder ihrer Masse unterliegen, zusammen mit dem bekannten Volumenstrom des zugeführten technischen Sauerstoffs dazu verwendet, die unbekannten Gasvolumenströme mittels eines Gleichungssystems aufgestellt wie folgt

$$k_{N_2, techn. Sauerstoff} \cdot Vol_{techn. Sauerstoff} + k_{N_2, Falschluft} \cdot Vol_{Falschluft} = k_{N_2, Abgas} \cdot Vol_{Abgas}$$

$$k_{Ar, techn. Sauerstoff} \cdot Vol_{techn. Sauerstoff} + k_{Ar, Falschluft} \cdot Vol_{Falschluft} = k_{Ar, Abgast} \cdot Vol_{Abgas}$$

mit

$k_{N_2.techn.Sauerstoff} \approx$     Konzentration (in %) des Stickstoffs ($N_2$) in dem, dem Prozeß zuströmenden technischen Sauerstoff

$k_{Ar,techn.Sauerstoff} \approx$     Konzentration (in %) des Argons (Ar) in dem, dem Prozeß zuströmenden technischen Sauerstoff

$k_{N_2,Falschluft} \approx$     Konzentration (in %) des Stickstoffs ($N_2$) in der, dem Prozeß zuströmenden Falschluft

$k_{Ar,Falschluft} \approx$     Konzentration (in %) des Argons (Ar) in der, dem Prozeß zuströmenden Falschluft

$Vol_{techn.Sauerstoff} \approx$     Gasvolumenstrom des, dem Prozeß zuströmenden technischen Sauerstoffs

$Vol_{Falschluft} \approx$     Gasvolumenstrom der, dem Prozeß zuströmenden Falschluft

$Vol_{Abgas} \approx$     Gasvolumenstrom des vom Prozeß abströmenden Abgas

zu bestimmen, wobei hier die Größen des Gasvolumenstroms des technischen Sauerstoffs und die Konzentrationen des Stickstoffs ($N_2$) und des Argons (Ar) in den Gasvolumenströmen des technischen Sauerstoffs und der Falschluft; wie auch im Gasvolumenstrom des Abgases erfaßbar oder ermittelbar sein müssen, damit das o.a. Gleichungssystem vollständig nach den gesuchten unbekannten Größen, nämlich dem Gasvolumenstrom der Falschluft und dem Gasvolumenstrom des Abgases hin aufgelöst werden kann. Die Auflösung dieses Gleichungssystems ergibt dann für die gesuchten Größen

$$Vol_{Abgas} = \left( \frac{k_{Ar,techn.Sauerstoff} - \frac{k_{Ar,Falschluft}}{k_{N_2,Falschluft}} \cdot k_{N_2,techn.Sauerstoff}}{k_{Ar,Abgas} - \frac{k_{Ar,Falschluft}}{k_{N_2,Falschluft}} \cdot k_{N_2,Abgas}} \right) \cdot Vol_{techn.Sauerstoff}$$

und

$$Vol_{Falschluft} = \frac{Vol_{Abgas} \cdot k_{N_2,Abgas} - Vol_{techn.Sauerstoff} \cdot k_{N_2,techn.Sauerstoff}}{k_{N_2,Falschluft}}$$

Eine weitere Ausführungsform des Verfahrens nach der vorliegenden Erfindung ist dadurch gekennzeichnet, daß als Bestimmungsgase (Tracer) zwei-Gase, nämlich das Edelgas Helium (He) und das Edelgas Argon (Ar), in bekannter oder zu messender Konzentration in den Gasen, die dem Prozeß zuströmen, nämlich in dem. technischen Sauerstoff und in der Falschluft vorhanden sind oder diesen zugesetzt werden und die selbst durch den Prozeß keiner Veränderung, insbesondere keiner Verminderung oder Erhöhung ihres Volumens oder ihrer Masse unterliegen, zusammen mit dem bekannten Volumenslrom des zugeführten technischen Sauerstoffs dazu verwendet werden die unbekannten Gasvolumenströme mittels eines Gleichungssystems aufgestellt wie folgt

$$k_{He, techn. Sauerstoff} \cdot Vol_{techn. Sauerstoff} + k_{He, Falschluft} \cdot Vol_{Falschluft} = k_{He, Abgas} \cdot Vol_{Abgas}$$

$$k_{Ar,techn.Sauerstoff} \cdot Vol_{techn. Sauerstoff} + k_{Ar, Falschluft} \cdot Vol_{Falschluft} = k_{Ar,Abgas} \cdot Vol_{Abgas}$$

mit:

$k_{He,techn.Sauerstoff} \approx$     Konzentration (in %) des Heliums (He) in dem, dem Prozeß zuströmenden technischen Sauerstoff

$k_{Ar,techn.Sauerstoff} \approx$     Konzentration (in %) des Argons (Ar) in dem, dem Prozeß zuströmenden technischen Sauerstoff

$k_{He,Falsehluft} \approx$       Konzentration (in %) des Heliums (He) in der, dem Prozeß zuströmenden Falschluft

$k_{Ar,Falschluft} \approx$       Konzentration (in %) des Argons (Ar) in der, dem Prozeß zuströmenden Falschluft

$Vol_{techn.Sauerstoff} \approx$       Gasvolumenstrom des, dem Prozeß zuströmenden technischen Sauerstoffs

$Vol_{Falschluft} \approx$       Gasvolumenstrom der, dem Prozeß zuströmenden Falschluft

$Vol_{Abgas} \approx$       Gasvolumenstrom des vom Prozeß abströmenden Abgas

zu bestimmen, wobei hier die Größen des Gasvolumenstroms des technischen Sauerstoffs und die Konzentrationen des Heliums (He) und des Argons (Ar) in den Gasvolumenströmen des technischen Sauerstoffs und der Falschluft, wie auch im Gasvolumenstrom des Abgases erfaßbar oder ermittelbar sein müssen, damit das o.a. Gleichungssystem vollständig nach den gesuchten unbekannten Größen, nämlich dem Gasvolumenstrom der Falschluft und dem Gasvolumenstrom des Abgases hin aufgelöst werden kann. Die Auflösung dieses Gleichungssystems ergibt dann für die gesuchten Größen.

$$Vol_{Abgas} = \left( \frac{k_{Ar,techn.Sauerstoff} - \dfrac{k_{Ar,Falschluft}}{k_{He,Falschluft}} \cdot k_{He,techn.Sauerstoff}}{k_{Ar,Abgas} - \dfrac{k_{Ar,Falschluft}}{k_{He,Falschluft}} \cdot k_{He,Abgas}} \right) \cdot Vol_{techn.Sauerstoff}$$

und

$$Vol_{Falschluft} = \frac{Vol_{Abgas} \cdot k_{He,Abgas} - Vol_{techn.Sauerstoff} \cdot k_{He,techn.Sauerstoff}}{k_{He,Falschluft}}$$

Vorzugsweise kann bei dem Verfahren nach der vorliegenden Erfindung dem Prozeß zusätzlich ein Kohlenstoff (C)/Stickstoff($N_2$) - Gemisch zugeführt werden, wobei von diesem Gemisch sowohl der Gasvolumenstrom, als auch die jeweiligen Konzentrationen des Kohlenstoffs (C), wie des Stickstoffs($N_2$) in diesem Gemisch bekannt bzw. meßbar sind und diese Daten dann zur Bestimmung der unbekannten Gasvolumenströme mit in die aufgestellten Gleichungen eingehen müssen,

Bei dem Verfahren nach der vorliegenden Erfindung wird als technischer Sauerstoff ein Gemisch verwendet, welches aufgrund seines Herstellungsverfahrens, nämlich mittels der Vakuum-Absorptionstechnik (z.B. VSA oder VSPA-Verfahren der Firma Air Liquide) bereits einen bestimmten Anteil an den Gasen Argon, Helium und Stickstoff aufweist, die dann nicht mehr gesondert zugesetzt werden müssen. So ist beispielsweise die Zusammensetzung nach Volumenanteilen des technischen Sauerstoffs, der mit einer VSA-Anlage der Firma Air Liquide hergestellt wid, wie folgt:

93 % $O_2$,
4,5 % Ar,
2,5 % $N_2$
ca. 0,00008 % He (= 8ppm He) und weitere
Spuren von $H_2O$, $CO_2$ und $C_nH_n$.

Es erübrigt sich also im Falle des Betriebes einer solchen Sauerstoffanlage besondere Bestimmungsgase (Tracer) gesondert zuzusetzen, da sie bei einem derartigen Sauerstoffherstellungsverfahren ohnehin mit anfallen.

Der Vorteil des Verfahrens nach der vorliegenden Erfindung besteht wesentlich darin, daß die im Wege des erfindungsgemäßen Verfahrens bestimmten Gasvolumenströme in ein mathematisches Modell des Prozesses einfließen können, um mit Hilfe dieses Modells den Prozeß sodann besser steuern bzw. regeln zu können.

So können nach Bestimmung des Abgasvolumenstromes im Elektroofen die Durchflußmengen an CO, $H_2$, und weiteren Komponenten berechnet werden. Ebenso kann mit Hilfe der Kenntnis des Abgasvolumenstromes die notwendige Sauerstoffrate zur vollständigen Oxidation von diesen Gasen ermittelt werden. Hierdurch ist es möglich zu jedem-Zeitpunkt eine optimale Menge an Sauerstoff für die Oxidation von CO und $H_2$ zur Verfügung zu stellen.

Für die Energieoptimierung des Ofens ist darüberhinaus der Falschluftvolumenstrom von großer Bedeutung. Die Entkohlungsgeschwindigkeit des Bades kann hierüber bestimmt und die Stoffströme von CO, $CO_2$ und $O_2$ können berechnet werden und in ein Prozeßmodell eingehen.

Ein weiterer enormer Kostenvorteil des Verfahrens nach der vorliegenden Erfindung besteht in der Verwendung eines technischen Sauerstoffs, der bereits alle für das Verfahren nach der vorliegenden Erfindung benötigten Bestimmungsgase (Tracer) enthält, ein technischer Sauerstoff wie er bereits bei Verwendung eines Sauerstoffherstellungsverfahrens nach der Vakuum Absorptionsmethode entsteht Das gesonderte Zusetzen von einem Tracer wie Argon oder Helium würde die stahlerzeugenden Kosten hingegen um ca. 2,- DM bis 10,- DM/t Flüssigsfahl erhöhen. Diese Kosten sind in vielen Fällen jedoch nur schwer tragbar, so daß das erfindungsgemäße Verfahren neben dem enormen technischen Vorzug auch noch zu einem bedeutenden wirtschaftlichen Vorteil führt

Im folgenden werden nicht einschränkend zu verstehende Ausführungsbeispiele anhand der Zeichnung besprochen. In dieser zeigen:

Fig.1    eine schematische Darstellung eines Elektroofens zur Stahlerzeugung mit Meßsonden für ein Massenspektrometer zur Abgasanalyse am Krümmer des Ofens,

Fig. 2    eine schematische Darstellung eines Prozesses der Flüssigphase mit zuströmenden und abströmenden Gasen,

Fig. 3    eine schematische Darstellung eines stahlerzeugenden Prozesses im Elektroofen mit zuströmendem technischen Sauerstoff und zuströmender Falschluft bestimmter Zusammensetzung und abströmendem Abgas, und

Fig. 4    eine schematische Darstellung eines stahlerzeugenden Prozesses im Elektroofen mit zuströmendem technischen Sauerstoff und zuströmender Falschluft einer weiteren bestimmten Zusammensetzung und abströmendem Abgas.

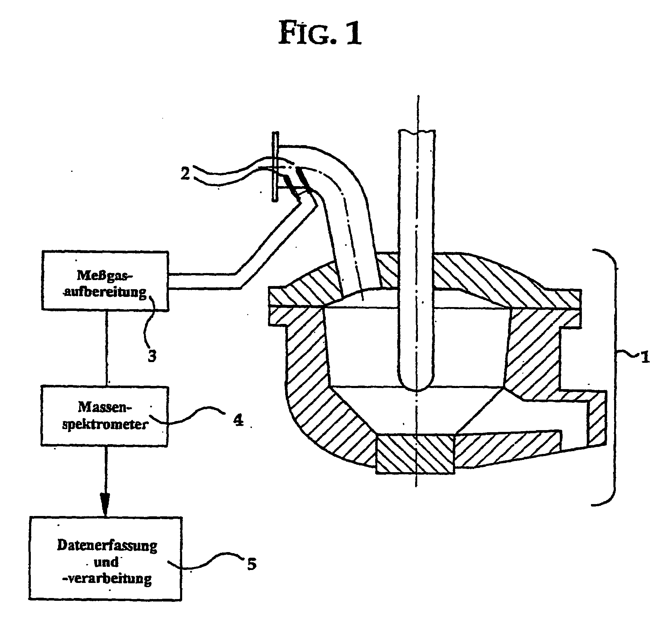

Fig. 1 zeigt eine schematische Darstellung eines Elektroofens **1** zur Stahlerzeugung mit Meßsonden **2** für ein Massenspektrometer **4** zur Abgasanalyse am Krümmer des Ofens. Die erfaßten Meßwerte werden zunächst in einer Meßgasaufbereitung **3** meßtechnisch aufbereitet, um dann an das Massenspektrometer **4** übertragen zu werden. Die Analyseergebnisse des Massenspektrometers **4** bilden dann schließlich die Eingangsgrößen einer Datenerfassung und Datenverarbeitung **5**, die zur Prozeßsteuerung eingesetzt werden kann.

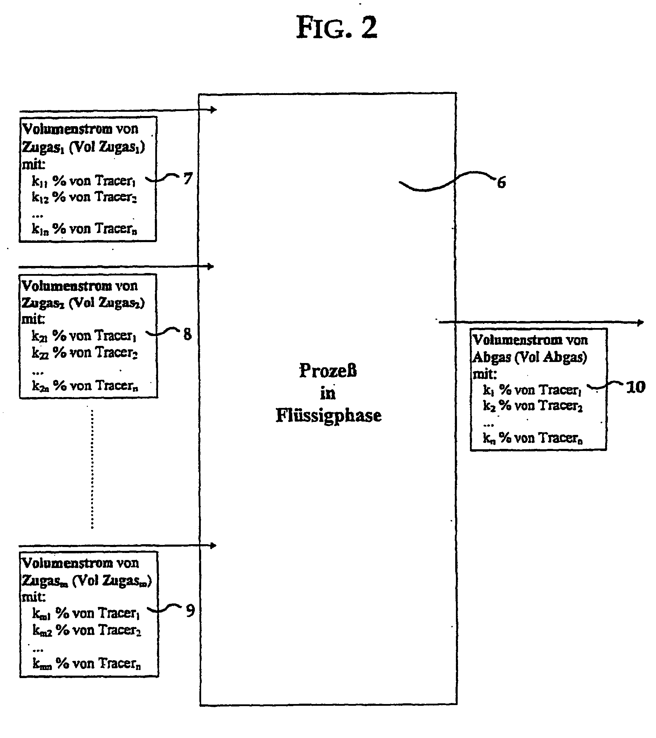

Fig. 2 zeigt eine eine schematische Darstellung eines Prozesses der Flüssigphase **6** mit zuströmenden und abströmenden Gasen. Hier strömen die Gase $\text{Zugas}_1$ 7, $\text{Zu-. gas}_2$ **8** bis $\text{Zugas}_m$ **9**, also insgesamt m Gase dem Prozeß zu und ein Abgas 10 vom Prozeß ab. Jedes der Gase enthält eine bestimmte Konzentration der n verschiedenen Bestimmungsgase (Tracer), wobei es sich bei diesen Bestimmungsgasen um Gase handelt, die keiner Veränderung durch den Prozeß 6 unterliegen, vorzugsweise Inertgase wie $N_2$, Helium oder ähnliches. Die Messung der Konzentration dieser Tracer im Volumenstrom des Abgases gibt dann unter der Voraussetzung, daß genügend weitere Daten bekannt, sind Aufschluß über nicht direkt meßbare Größen wie beispielsweise den Volumenstrom des Abgases. Dies ist aufgrund einer Bilanz der Volumenströme möglich, die die Unveränderlichkeit der Konzentration der Tracer durch den Prozeß 6 berücksichtigt. Das Gleichungssystem, das diese Bilanz beschreibt lautet

$$
\begin{aligned}
k_{tracer_1,Zugas_1} \cdot Vol_{Zugas_1} &+ \cdots + k_{tracer_1,Zugas_m} \cdot Vol_{Zugas_m} = k_{tracer_1,Abgas} \cdot Vol_{Abgas} \\
k_{tracer_2,Zugas_1} \cdot Vol_{Zugas_1} &+ \cdots + k_{tracer_2,Zugas_m} \cdot Vol_{Zugas_m} = k_{tracer_2,Abgas} \cdot Vol_{Abgas} \\
\vdots \quad &+ \cdots + \vdots \qquad\qquad = \quad \vdots \\
k_{tracer_n,Zugas_1} \cdot Vol_{Zugas_1} &+ \cdots + k_{tracer_n,Zugas_m} \cdot Vol_{Zugas_m} = k_{tracer_n,Abgas} \cdot Vol_{Abgas}
\end{aligned}
$$

Sind hierin nun genügend Größen bekannt, so kann es nach den unbekannten gesuchten Größen hin aufgelöst werden, wodurch diese indirekt meßbar werden.

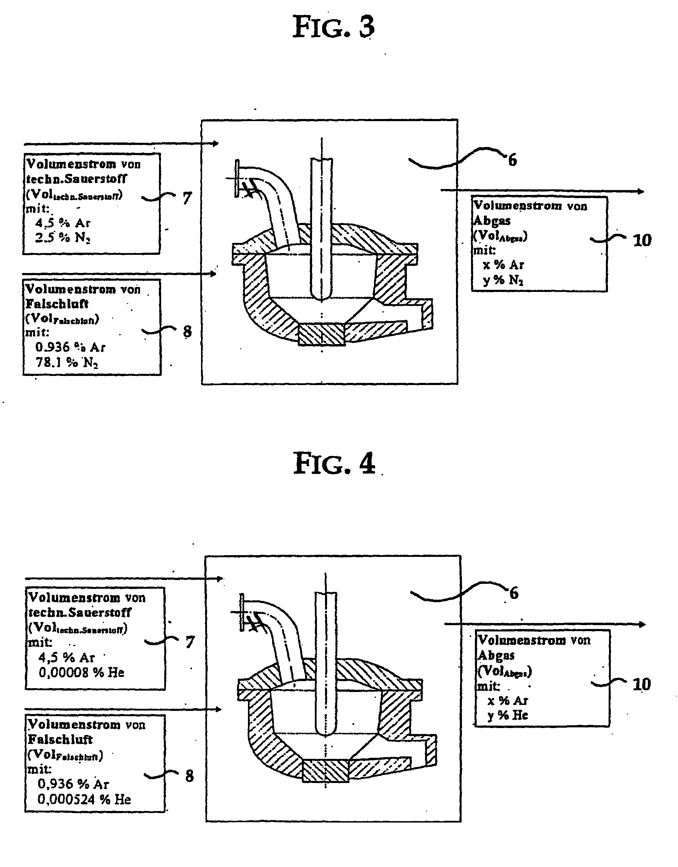

Fig. 3 zeigt eine schematische Darstellung eines stahlerzeugenden Prozesses im Elektroofen 6 mit zuströmendem technischen Sauerstoff als erstem zuströmenden Gas 7, zuströmender Falschluft als zweitem zuströmendem Gas 8 und abströmendem Abgas 10. Der technische Sauerstoff weist hierbei folgende Konzentrationen der Tracer auf:

4,5 % Ar und 2,5 % $N_2$

Für die vom Prozeß 6 infolge von Undichtigkeiten des Ofens angesaugte Falschluft lauten diese Daten:

0,936 % Ar und 78,1 % $N_2$

Das Gleichungssystem für die Volumenstrombilanz ergibt sich in diesem Falle somit zu:

$$0{,}025 \cdot Vol_{techn.\ Sauerstoff} + 0{,}781 \cdot Vol_{Falschluft} = x \cdot Vol_{Abgas}$$

$$0{,}045 \cdot Vol_{techn.\ Sauerstoff} + 0{,}00936 \cdot Vol_{Falschluft} = y \cdot Vol_{Abgas}$$

Der Volumenstrom des technischen Sauerstoffs kann hier direkt gemessen werden. Ebenso können die Konzentrationen von Argon und Stickstoff ($N_2$) im Abgasvolumenstrom mit Hilfe des Massenspektromebers bestimmt werden. Gesucht sind im hier vorliegenden Falle jedoch der Volumenstrom des Abgases, wie der der Falschluft. Hierfür ergeben sich nach der obigen Gleichung somit schließlich:

$$Vol_{Abgas} = \frac{0{,}04470038}{x{-}0{,}01198464 \cdot y} \cdot Vol_{tech.\ Sauerstoff}$$

und

$$Vol_{Falschluft} = \frac{Vol_{Abgas} \cdot y - Vol_{techn.Sauerstoff} \cdot 0{,}025}{0.781}$$

Fig. 4 zeigt eine schematische Darstellung eines stahlerzeugenden Prozesses im Elektroofen 6 mit zuströmendem technischen Sauerstoff als erstem zuströmenden Gas 7, zuströmender Falschluft als zweitem zuströmendem Gas 8 und abströmendem Abgas 10. Der technische Sauerstoff weist hierbei folgende Konzentrationen der Tracer auf:

4,5 % Ar und 0,00008 % He (= 0,8 ppm)

Für die vom Prozeß 6 infolge von Undichtigkeiten des Ofens angesaugte Falschluft laulen diese Daten:

0,936 % Ar und 0,000524 % He (= 5,24 ppm)

Das Gleichungssystem für die Volumenstrombilanz ergibt sich in diesem Falle somit zu:

$$0{,}0000008 \cdot Vol_{techn.\ Sauerstoff} + 0{,}00000524 \cdot Vol_{Falschluft} = x \cdot Vol_{Abgas}$$

$$0{,}045 \cdot Vol_{techn.\ Sauerstoff} + 0{,}00936 \cdot Vol_{Falschluft} = y \cdot Vol_{Abgas}$$

Der Volumenstrom des technischen Sauerstoffs kann hier direkt gemessen werden. Ebenso können die Konzentrationen von Argon und Stickstoff ($N_2$) im Abgasvolumenstrom mit Hilfe des Massenspektrometers bestimm werden. Gesucht sind im hier vorliegenden Falle jedoch der Volumenstrom des Abgases, wie der der Falschluft Hierfür ergeben sich nach der obigen Gleichungsomit schließlich:

$$Vol_{Abgas} = \frac{0{,}04357099}{x{-}1786{,}25954 \cdot y} \cdot Vol_{techn.\ Sauerstoff}$$

und

$$Vol_{Falschluft} = \frac{Vol_{Abgas} \cdot y - Vol_{techn.\ Sauerstoff} - 0{,}0000008}{0{,}00000524}$$

## Patentansprüche

1. Verfahren zur Bestimmung von direkter Messung nicht, oder nicht leicht zugänglicher Gasvolumenströme zu und von stahlerzeugenden Schmelzprozessen in einem Elektroofen, der Undichtigkeiten aufweist, bei dem

a) technischer Sauerstoff mittels Vakuum-Adsorptionstechnik hergestellt wird, der aufgrund seines Herstellungsverfahrens bereits einen Anteil an dan Bestimmungsgasen, Sogenannten Tracern, Argon, Helium und Stickstoff aufweist,

b) im Elektroofen ein stahlerzeugender Schmelzprozeß durchgeführt, wird und dem Schmelzprozeß einerseits dieser technische Sauerstoff mit einem quantitativ bekannten Gasvolumenstrom gewollt zugeführt wird und andererseits durch die Undichtigkeiten Umgebungsluft als sogenannte Falschluft mit quantitativ unbekanntem Gasvolumenstrom zuströmt,

c) von dem Elektroofen durch einen Abgaskrümmer ein Abgas mit einem quantitativ unbekannten Gasvolumenstrom abströmt und

d) mindestens zwei der drei Bestimmungsgase, die in bekannter Konzentration in dem technischen Sauerstoff und in der Falschluft vorhanden sind und selbst durch den im Elektroofen stattfindenden Schmelzprozess keiner Veränderung, insbesondere keiner Verminderung oder Erhöhung ihres' Volumens oder ihrer Masse unterliegen, dazu verwendet werden, die unbekannten Gasvolumenströme der Falschluft und des Abgases mittels eines Gleichungssystems aufgestellt wie folgt:

$$k_{tracer1,techn.Saucrstoff} \cdot Vol_{techn.Sauerstoff} + k_{tracer1,Falschluft} \cdot Vol_{Falschluft}$$

$$= k_{tracer1,Abgas} \cdot Vol_{Abgas}$$

$$k_{tracer2,techn.Sauerstoff} \cdot Vol_{techn.Sauerstoff} + k_{tracer2,Falschluft} \cdot Vol_{Falschluft}$$

$$= k_{tracer2,Abgas} \cdot Vol_{Abgas}$$

mit den folgenden, bekannten Grössen:

$k_{tracer1,techn.Sauerstoff} \approx$     Konzentration (in %) des Tracers 1 in dem dem Prozeß zuströmenden technischen Sauerstoff,

$k_{tracer2,techn.Sauerstoff} \approx$     Konzentration (in %) des Tracers 2 in dem dem Prozeß zuströmenden technischen Sauerstoff,

$k_{tracer1,Falschluft} \approx$     Konzentration (in %) des Tracers 1 in der dem Prozeß zuströmenden Falschluft,

$k_{tracer2,Falschluft} \approx$     Konzentration (in %) des Tracers 2 in der dem Prozeß zuströmenden Falschluft und

$Vol_{techn.Sauerstoff} \approx$     Gasvolumenstrom des dem Prozeß zuströmenden technischen Sauerstoffs,

mit den folgenden, im Elektroofen zu bestimmenden Grössen:

$k_{tracer1,Abgas} \approx$     Konzentration (in %) des Tracers 1 im Abgas,

$k_{tracer2,Abgas} \approx$     Konzentration (in %) des Tracers 2 im Abgas,

$Vol_{Falschluft} \approx$     Gasvolumenstrom der dem Prozeß zuströmenden Falschluft und

$Vol_{Abgas} \approx$     Gasvolumenstrom des vom Prozeß abströmenden Abgases

zu bestimmen, wobei das o.g. Gleichungssystem nach den gesuchten unbekannten Grössen, nämlich Gasvolumenstrom der Falschluft und dem Gasvolumenstrom des Abgases, aufgelöst wird.

2. Verfahren nach Anspruch 1, **dadurch gekennzeichnet, dass** zur Messung der Bestimmungsgase (Tracer) in den dem Prozess zuströmenden oder von diesem abströmenden Gasen ein Massenspektrometer benutzt wird.

3. Verfahren nach Anspruch 1, **dadurch gekennzeichnet, dass** die Messung der Konzentration der Bestimmungsgase im Volumenstrom des Abgases im Abgaskrümmer erfolgt.

4. Verfahren nach Anspruch 1, **dadurch gekennzeichnet, dass** der technische Sauerstoff nach dem VSA oder dem VPSA Verfahren hergestellt ist.

## Claims

1. Method for the determination of volumetric gas flows which are inaccessible to direct measurement or difficult to access for direct measurement purposes, into and out of steel-producing melting processes in an electric furnace having leakages in which method

   a) tonnage oxygen is produced by means of the vacuum adsorption method, which tonnage oxygen as a result of its method of production already contains certain levels of the determining gases, so called tracers, argon, helium and nitrogen,

   b) a steel-producing melting process is performed in the electric furnace and on one hand tonnage oxygen as an intended inflow in the form of a quantitatively known volumetric flow rate and on the other hand through the leakages ambient air as so called infiltrated air in an unknown volumetric gas flow flow into the melting process,

   c) a waste gas in an unknown volumetric gas flow flows out of the electric furnace through a waste gas pipe bend, and

   d) at least two of the three determining gases, which are present in known concentration in the tonnage oxygen and in the infiltrated air and which are not themselves subject to any changes as a result of the melting process taking place in electric furnace, in particular with regard to reductions or increases in their volume or mass, are employed to determine the unknown volumetric gas flows of infiltrated air and waste gas, by means of a system of equations constructed as follows:

   $$k_{tracer1,techn.Sauerstoff} \cdot Vol_{techn.Sauerstoff} + k_{tracer1,Faischluft} \cdot Vol_{Falschluft} = k_{tracer1,Abgas} \cdot Vol_{Abgas}$$

   $$k_{tracer2,techn.Sauerstoff} \cdot Vol_{techn.Sauerstoff} + k_{tracer2,Falschluft} \cdot Vol_{Falschluft} = k_{tracer2,Abgas} \cdot Vol_{Abgas}$$

   with the following known quantities:

   | | |
   |---|---|
   | $k_{tracer1,techn.Sauerstoff} \approx$ | concentration (in %) of tracer 1 contained in the tonnage oxygen flowing into the process, |
   | $k_{tracer2,techn.Sauerstoff} \approx$ | concentration (in %) of tracer 2 contained in the tonnage oxygen flowing into the process, |
   | $k_{tracer1,Falschluft} \approx$ | concentration (in %) of tracer 1 in the infiltrated air flowing into the process, |
   | $k_{tracer2,Falschluft} \approx$ | concentration (in %) of tracer 2 contained in the infiltrated air flowing into the process and |
   | $Vol_{techn.Sauerstoff} \approx$ | volumetric gas flow of the tonnage oxygen flowing into the process, |

   with the following quantities to be determined in the electric furnace:

   | | |
   |---|---|
   | $k_{tracer1,Abgas} \approx$ | concentration (in %) of tracer 1 contained in the waste gas, |
   | $k_{tracer2,Abgas} \approx$ | concentration (in %) of tracer 2 contained in the waste gas, |
   | $Vol_{Falschluft} \approx$ | volumetric gas flow of the infiltrated air flowing into the process and |
   | $Vol_{Abgas} \approx$ | volumetric gas flow of the waste gas flowing out of the process, |

   whereby the above-stated system of equations is solved completely with regard to the sought, unknown quantities, namely the volumetric gas flow of infiltrated air and the volumetric gas flow of waste gas.

2. Method in accordance with Claim 1, **characterised in that** a mass spectrometer is used to measure the determining gases (tracers) in the gases flowing into and out of the process.

3. Method in accordance with Claim 1, **characterised in that** the measurement of the concentration of the determining gases in the volumetric flow rate of the waste gas takes place in a waste gas pipe bend.

4. Method in accordance with Claim 1, **characterised in that** the tonnage oxygen is produced by means of the VSA or VSPA method.

**Revendications**

1. Procédé pour déterminer des flux de volumes de gaz inaccessibles au mesurage direct ou difficilement accessibles au mesurage direct, vers des et provenant de processus de fusion produisant de l'acier dans un four électrique qui présente des fuites, dans lequel

   a) de l'oxygène technique est produit au moyen de la technique d'adsorption à vide, qui - en raison de son procédé de production - présente déjà une part des gaz de détermination, ce que l'on appelle les traceurs (tracer), à savoir l'argon, l'hélium et l'azote,

   b) un processus de fusion produisant de l'acier est mis en oeuvre dans le four électrique, et, d'un côté, cet oxygène technique avec un flux de volume de gaz quantitativement connu est amené de manière voulue au processus de fusion et, de l'autre côté, de l'air ambiant comme ce que l'on appelle l'air entré accidentellement avec un flux de volume de gaz quantitativement inconnu afflue à travers les fuites,

   c) un gaz d'échappement avec un flux de volume de gaz quantitativement inconnu s'échappe du four électrique à travers un coude d'échappement, et

   d) au moins deux des trois gaz de détermination qui sont présents en concentration connue dans l'oxygène technique et dans l'air entré accidentellement et qui, eux mêmes, ne sont soumis à aucun changement, en particulier à aucune diminution ou augmentation de leur volume ou de leur masse par le processus de fusion ayant lieu dans le four électrique, sont utilisés pour déterminer les flux de volumes de gaz inconnus de l'air entré accidentellement et du gaz d'échappement au moyen d'un système d'équations établi comme suit :

   $$k_{tracer1,techn.Sauerstoff} \cdot Vol_{techn.Sauerstoff} + k_{tracer1,Falschluft} \cdot Vol_{Falschluft}$$

   $$= k_{tracer1,Abgas} \cdot Vol_{Abgas}$$

   $$k_{tracer2,techn.Sauerstoff} \cdot Vol_{techn.Sauerstoff} + k_{tracer2,Falschluft} \cdot Vol_{Falschluft}$$

   $$= k_{tracer2,Abgas} \cdot Vol_{Abgas}$$

   avec les grandeurs connues suivantes:

   | | |
   |---|---|
   | $k_{tracer1,techn.Sauerstoff} \approx$ | concentration (en %) du traceur 1 dans l'oxygène technique affluant au processus, |
   | $k_{tracer2,techn.Sauerstoff} \approx$ | concentration (en %) du traceur 2 dans l'oxygène technique affluant au processus, |
   | $k_{tracer1,Falschluft} \approx$ | concentration (en %) du traceur 1 dans l'air entré accidentellement affluant au processus, |
   | $k_{tracer2,Falschluft} \approx$ | concentration (en %) du traceur 2 dans l'air entré accidentellement affluant au processus, et |
   | $Vol_{techn.Sauerstoff} \approx$ | flux de volume de gaz de l'oxygène technique affluant au processus, |

   avec les grandeurs suivantes à déterminer dans le four électrique :

   | | |
   |---|---|
   | $k_{tracer1,Abgas} \approx$ | concentration (en %) du traceur 1 dans le gaz d'échappement, |
   | $k_{tracer2,Abgas} \approx$ | concentration (en %) du traceur 2 dans le g gaz d'échappement, |
   | $Vol_{Falschluft} \approx$ | flux de volume de gaz de l'air entré accidentellement affluant au processus, et |
   | $Vol_{Abgas} \approx$ | flux de volume de gaz du gaz d'échappement quittant le processus |

   le système d'équations mentionné ci-dessus étant résolu à l'égard des grandeurs inconnues cherchées, à savoir du flux de volume de gaz de l'air entré accidentellement et du flux de volume de gaz du gaz d'échappement.

2. Procédé selon la revendication 1, **caractérisé par le fait que**, pour mesurer les gaz de détermination (traceurs) dans les gaz affluant au processus ou quittant celui-ci, on utilise un spectromètre de masse.

3. Procédé selon la revendication 1, **caractérisé par le fait que** le mesurage de la concentration des gaz de détermination dans le flux de volume du gaz d'échappement se fait dans le coude d'échappement.

**4.** Procédé selon la revendication 1, **caractérisé par le fait que** l'oxygène technique est produit selon le procédé VSA ou selon le procédé VPSA.

# FIG. 1

# FIG. 2

Volumenstrom von
Zugas$_1$ (Vol Zugas$_1$)
mit:
  $k_{11}$ % von Tracer$_1$    7
  $k_{12}$ % von Tracer$_2$
  ...
  $k_{1n}$ % von Tracer$_n$

Volumenstrom von
Zugas$_2$ (Vol Zugas$_2$)
mit:
  $k_{21}$ % von Tracer$_1$    8
  $k_{22}$ % von Tracer$_2$
  ...
  $k_{2n}$ % von Tracer$_n$

Volumenstrom von
Zugas$_m$ (Vol Zugas$_m$)
mit:
  $k_{m1}$ % von Tracer$_1$    9
  $k_{m2}$ % von Tracer$_2$
  ...
  $k_{mn}$ % von Tracer$_n$

Prozeß
in
Flüssigphase

6

Volumenstrom von
Abgas (Vol Abgas)
mit:
  $k_1$ % von Tracer$_1$    10
  $k_2$ % von Tracer$_2$
  ...
  $k_n$ % von Tracer$_n$

# FIG. 3

Volumenstrom von
techn. Sauerstoff
($Vol_{techn.Sauerstoff}$)
mit:
  4,5 % Ar
  2,5 % $N_2$

~ 7

Volumenstrom von
Falschluft
($Vol_{Falschluft}$)
mit:
  0,936 % Ar
  78,1 % $N_2$

~ 8

6

Volumenstrom von
Abgas
($Vol_{Abgas}$)
mit:
  x % Ar
  y % $N_2$

~ 10

# FIG. 4

Volumenstrom von
techn. Sauerstoff
($Vol_{techn.Sauerstoff}$)
mit:
  4,5 % Ar
  0,00008 % He

~ 7

Volumenstrom von
Falschluft
($Vol_{Falschluft}$)
mit:
  0,936 % Ar
  0,000524 % He

~ 8

6

Volumenstrom von
Abgas
($Vol_{Abgas}$)
mit:
  x % Ar
  y % He

~ 10